# EUROPÄISCHE PATENTSCHRIFT

(11) **EP 2 977 031 B1**
(45) Veröffentlichungstag und Bekanntmachung des Hinweises auf die Patenterteilung: **17.01.2018**
(21) Anmeldenummer: 15001725.9
(22) Anmeldetag: 11.06.2015
(51) Int. Cl.: A61F 5/01, A61F 13/10, A61F 5/30

(54) **EPICONDYLITISSPANGE**
EPICONDYLITIS BRACE
ORTHÈSE ÉPICONDYLIENNE

(30) Priorität: 16.07.2014 DE 202014005803 U
(43) Veröffentlichungstag der Anmeldung: 27.01.2016
(73) Patentinhaber: Thuasne Deutschland GmbH, 30938 Burgwedel (DE)
(72) Erfinder: Franciscy, Sabine, 30655 Hannover (DE); Fritzsche, Stefan, 27308 Kirchlinteln (DE); Siedentop, Tjark, 38106 Braunschweig (DE); Schneider-Nieskens, Reinhold, 29223 Celle (DE)
(74) Vertreter: Siekmann, Gunnar

(56) Entgegenhaltungen:
- EP-A1- 2 184 040
- US-A- 4 182 318

## Beschreibung

Die Erfindung betrifft eine Epicondylitisspange zur Anlage an einen Unterarm eines Patienten, aufweisend ein zwischen einem ersten und einem zweiten Ende gekrümmtes Formteil mit einer konkaven Anlageseite, ein an dem Formteil angebrachtes Befestigungsmittel zum Anlegen der Epicondylitisspange und eine an der Anlageseite angeordnete Einlage mit wenigstens einer in die Einlage eingearbeiteten Pelotte, wobei die Einlage lösbar an dem Formteil befestigt ist.

Eine Epicondylitisspange der eingangs genannten Art ist aus der EP2184040A1 bekannt. Diese Epicondylitisspange weist eine abnehmbare Pelotte auf, die so an einem Fassungsteil befestigt ist, dass das Fassungsmittel, sowohl am rechten als auch am linken Arm in jeweils der therapeutisch erforderlicher Lage der Pelotte sitzt.

Durch die in die Einlage eingearbeitete Pelotte kann mit einer nahe dem Ellenbogen an den Unterarm angelegten Epicondylitisspange gezielt Druck auf überlastete, gereizte und/oder entzündete Muskel- und Sehnenstränge des Unterarms ausgeübt werden. Die Muskel- und Sehnenstränge werden dabei entlastet, stabilisiert und beruhigt, so dass einerseits eine Heilung unterstützt und andererseits eine Schmerzlinderung der betroffenen Muskel- und Sehnenstränge erreicht ist.

Bekannten Epicondylitisspangen ist dabei gemein, dass diese ein ballenförmiges Druckpolster, die Pelotte, aufweisen, das direkt auf dem Muskel- und Sehnenansatz unterhalb des Ellenbogens positioniert wird. Die Einstellung des Drucks erfolgt dabei über das Befestigungsmittel, welches zumeist individuell einstellbar ist. Dieses Befestigungsmittel ermöglicht jedoch nur eingeschränkt eine punktuelle Erhöhung des Druckes auf die Schmerzstelle.
Aufgabe der Erfindung ist es, eine Epicondylitisspange dahingehend weiterzuentwickeln, dass diese auf einfache Weise individuell an einen Patienten anpassbar ist und einen für die Heilung optimalen Druck auf den Muskel- und Sehnenansatz des Unterarms am Ellenbogen ausübt.
Die Lösung dieser Aufgabe erfolgt mit einer Epicondylitisspange mit den Merkmalen des Schutzanspruchs 1. Weiterbildungen und vorteilhafte Ausgestaltungen der Erfindung sind in den nachgeordneten Schutzansprüchen angegeben.
Die Epicondylitisspange zur Anlage an einen Unterarm eines Patienten, aufweisend ein zwischen einem ersten und einem zweiten Ende gekrümmtes Formteil mit einer konkaven Anlageseite, ein an dem Formteil angebrachtes Befestigungsmittel zum Anlegen der Epicondylitisspange und eine an der Anlageseite angeordnete Einlage mit wenigstens einer in die Einlage eingearbeiteten Pelotte, zeichnet sich erfindungsgemäß dadurch aus, dass die Einlage lösbar an dem Formteil befestigt ist, und dass zwischen der Einlage und dem Formteil wenigstens ein Anlagebereich für wenigstens eine weitere Pelotte ausgebildet ist. Mit der zusätzlichen Pelotte, die in dem Anlagebereich angeordnet werden kann, lässt sich auf einfache Weise eine Druckerhöhung auf den Muskel- und Sehnenansatz des Unterarms eines zu behandelnden Patienten erreichen. Die Epicondylitisspange kann den Muskel- und Sehnenansatz unterhalb des Ellenbogens so auf optimale Weise stützen, wodurch die Wirkung der Epicondylitisspange auf den Muskel- und Sehnenansatz verbessert ist.

Auf einfache Weise sind die Einlage und das Formteil dabei über wenigstens einen Klettverschluss miteinander verbunden, der nahezu beliebig zu öffnen und zu schließen ist. Dieser Klettverschluss ist vorteilhafterweise in einem Randbereich des Formteils, der Einlage oder des Formteils und der Einlage angeordnet. Insbesondere bildet der Klettverschluss dabei einen wenigstens abschnittsweise umlaufenden Klettstreifen an der Anlageseite des Formteils aus. Um eine gleichstarke Haftung zwischen Einlage und Formteil zu erhalten, weist der Klettstreifen nach einer Weiterbildung eine gleichbleibende, insbesondere über seine gesamte Länge gleichbleibende, Breite auf. Bevorzugt weist der Klettstreifen eine Breite zwischen 0,3 cm und 1,5 cm, insbesondere zwischen 0,5 cm und 1,2 cm, insbesondere zwischen 0,7 cm und 0,9 cm, auf. Der Anlagebereich weist bevorzugt einen Durchmesser zwischen 2 cm und 6 cm, insbesondere zwischen 3 cm und 5 cm, auf. Der Anlagebereich weist damit einen Durchmesser auf, der wenigstens das Doppelte, insbesondere das Zweieinhalbfache der Breite des Streifens beträgt.

In einer bevorzugten Ausgestaltung ist der wenigstens eine Anlagebereich, insbesondere nur der wenigstens eine Anlagenbereich, für die wenigstens eine weitere Pelotte von dem als Klettverschluss ausgebildeten Randbereich eingefasst, so dass eine zusätzliche Pelotte zur Druckerhöhung zwischen der Einlage und dem Formteil platziert von diesen umschlossen ist, insbesondere vollständig umschlossen ist. Der Klettverschluss bildet dann eine Ringstruktur um den wenigstens einen Anlagebereich aus. Alternativ kann der Klettverschluss auch aus mehreren, verzweigten oder sich kreuzenden Klettstreifen bestehen.

Gemäß weiterer Ausgestaltungen ist vorgesehen, dass wenigstens eines der Enden den Anlagebereich aufweist, insbesondere, dass beide Enden je einen Anlagebereich aufweisen. Die an einen Unterarm angelegte Epicondylitisspange, erstreckt sich dann mit dem Formteil und der Einlage wenigstens zwischen zwei Bereichen des Unterarms, insbesondere zwei einander gegenüberliegenden Bereichen des Unterarms, auf die Druck ausgeübt werden soll. Dabei ermöglichen zwei oder mehr Anlagebereiche für Pelotten eine individuellere Anpassung der Epicondylitisspange an einen Patienten, in dem verschiedene, d. h. insbesondere verschieden große, unterschiedlich dicke oder verschieden große und unterschiedlich dicke, Pelotten in den Anlagebereichen angeordnet werden. Entsprechend den unterschiedlich großen Pelotten kann das erste Ende gemäß einer vorteilhaften Ausgestaltung auch einen größeren Anlagebereich als das zweite Ende aufweisen.

Ein ausreichender Halt der Einlage an dem Formteil kann dabei auch gewährleistet werden, wenn die Einlage und das Formteil nur an den Enden miteinander verbunden sind. Der Klettverschluss ist gemäß dieser Weiterbildung dann nicht vollständig umlaufend um einen äußeren Rand der Anlageseite sondern als ring-, u- oder hufeisenförmiger Streifen an den jeweiligen Enden, insbesondere an äußeren Rändern der Anlageseite an den jeweiligen Enden, angeordnet. Zwischen diesen Enden kann das Formteil tailliert ausgebildet sein, um eine anatomisch günstige Form zur Anlage der Epicondylitisspange nahe dem Ellenbogen, insbesondere in einem Abstand von 4 cm bis 5 cm von dem Ellenbogen, aufzuweisen.

In weiterer Ausgestaltung weisen die Anlageseite des Formteils und die Einlage zueinander kongruente Flächen auf. Die Einlage erstreckt sich dann mit Vorteil vollständig zwischen dem Formteil und einem Unterarm des Patienten, wodurch der Unterarm nicht direkt an dem Formteil anliegt und ein angenehmes Tragegefühl ermöglicht ist. Durch einen gegenüber dem Formteil überstehenden Rand, insbesondere wulstförmigen Rand, kann das angenehme Tragegefühl weiter optimiert sein, wobei die Einlage dann einen größeren Zuschnitt als die Anlageseite des Formteils aufweist. Die Einlage besteht dazu vorteilhafterweise aus einem weichen, flexiblen Material, insbesondere einem Stoff, insbesondere einem Filzstoff. Dieser kann zwischen den Enden, d. h. insbesondere in dem taillierten Bereich, eine gewellte oder gekerbte Oberflächenstruktur, insbesondere eine quer zu einem Krümmungsverlauf des Formteils gewellte oder gekerbte Oberfläche, aufweisen, um sich auf einfache Weise an die Krümmung des Formteils anzupassen und ungewollte Druckstellen am Unterarm des Patienten zu vermeiden.

Vorteilhaft gestaltet es sich zudem, wenn das Formteil wenigstens einen Anlagebereich aufweist, so dass der Anlagebereich eine feste Struktur mit einer glatten Oberfläche aufweist.

Um den Druck der Pelotten einer angelegten Epicondylitisspange auf den Muskel- und Sehnenansatz gezielt erhöhen zu können, ist vorgesehen, dass die in die Einlage eingearbeitete Pelotte an einem Ende der Einlage angeordnet ist und zusammen mit der in dem Anlagebereich positionierten Pelotte einen gemeinsamen Druckpunkt ausbildet. Je nach Größe, Dicke und Form der in dem Anlagebereich positionierten Pelotte wird der durch die standardmäßig in die Einlage eingearbeitete Pelotte auf einen Unterarm ausgeübte Druck verstärkt und individuell an den jeweiligen Patienten angepasst. Die den Enden zugeordneten Pelotten von Einlage und Anlagebereich weisen dabei vorteilhafterweise eine an die Breite und Flächenform der Enden, insbesondere der an den Enden ausgebildeten Anlagebereiche, angepasste Größe auf. Bevorzugt können die einen Druckpunkt ausbildenden Pelotten zudem gleichgroß sein, wobei zumindest die in die Einlage eingearbeitete Pelotte eine Größe und Form annähernd gleich, insbesondere kongruent zu dem Anlagebereich aufweist.
Als Befestigungsmittel kann ein außenseitig an dem Formteil angeordneter Klettverschluss vorgesehen sein. Dieser ermöglicht ebenso wie der Klettverschluss zwischen Einlage und Formteil ein nahezu beliebig häufiges Öffnen und Schließen und kann individuell festgezogen werden, um einen optimalen Sitz der Epicondylitisspange an dem Unterarm zu gewährleisten.

Weiter betrifft die Erfindung auch eine Pelotte, die sich dadurch auszeichnet, dass diese in den Anlagebereich der vorbezeichneten Epicondylitisspange einsetzbar ist. Bevorzugt ist diese Pelotte aus einem weichelastischem Elastomer gefertigt und weist blockende Eigenschaften für eine Anfangshaftung der Pelotte an dem Anlagebereich auf. Bevorzugte Materialien für das Elastomer sind Silikon oder ein TPE-Elastomer.

Um eine gute Anfangshaftung zu gewährleisten, ist weiterhin vorgesehen, dass die Pelotte eine an den Anlagebereich angepasste Form mit einer ebenen Anlagefläche aufweist und in Richtung eines zu behandelnden Unterarms konvex gewölbt ist. Durch die konvexe Wölbung erhöht sich der punktuell zu erzeugende Druck auf die Schmerzstelle ohne das sich unangenehm an dem Unterarm abzeichnende Kanten ausbilden.

Weiterhin kann sich die Pelotte dadurch auszeichnen, dass diese eine kreisförmige Anlagefläche aufweist oder in einer alternativen Ausgestaltung eine dreieckige Anlagefläche mit ausgerundeten Ecken aufweist.
Ein Ausführungsbeispiel der Erfindung ist in der Zeichnung dargestellt. Es zeigen:
- Figur 1:: eine erste perspektivische Ansicht einer erfindungsgemäßen Epicondylitisspange;
- Figur 2:: eine zweite perspektivische Ansicht der erfindungsgemäßen Epicondylitisspange gemäß Figur 1;
- Figur 3:: eine Draufsicht auf eine Einlage der erfindungsgemäßen Epicondylitisspange gemäß Figur 1 und Figur 2;
- Figur 4:: eine erste Detailansicht der erfindungsgemäßen Epicondylitisspange gemäß Figur 1 bis 3;
- Figur 5:: eine zweite Detailansicht der erfindungsgemäßen Epicondylitisspange gemäß Figur 1 bis 3;
- Figur 6:: eine dritte Detailansicht der erfindungsgemäßen Epicondylitisspange gemäß Figur 1 bis 3; und
- Figur 7:: eine vierte Detailansicht der erfindungsgemäßen Epicondylitisspange gemäß Figur 1 bis 3.

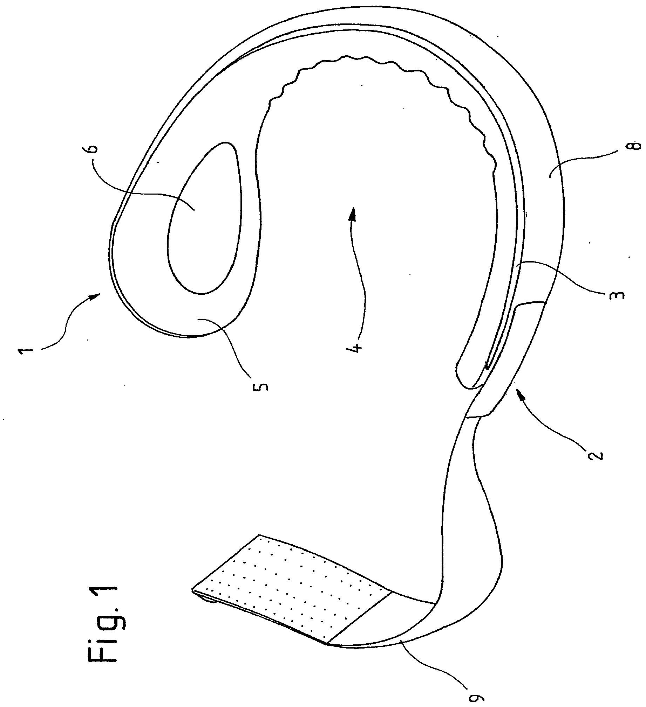

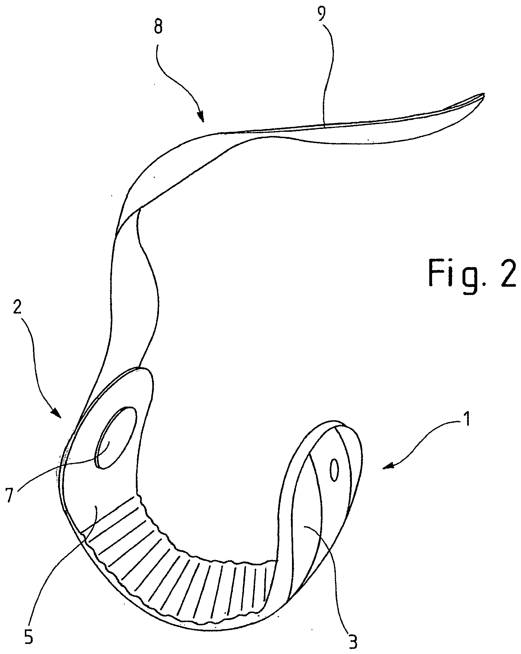

In Figur 1 und Figur 2 ist die Epicondylitisspange mit dem zwischen einem ersten Ende 1 und einem zweiten Ende 2 gekrümmten Formteil 3 dargestellt, das eine konkave Anlageseite 4 zur Anlage an einem Unterarm aufweist. An der Anlageseite 4 ist eine Einlage 5 befestigt, die aus einem Filzstoff besteht und sich über die gesamte Fläche der Anlageseite 4 erstreckt. In diese Einlage 5 sind zwei Pelotten 6, 7 eingearbeitet, von denen eine erste Pelotte 6 dem ersten Ende 1 und eine zweite Pelotte 7 dem zweiten Ende 2 zugeordnet ist. Die Pelotte 6 ist aus Figur 1 und die Pelotte 7 aus Figur 2 ersichtlich. Jede dieser Pelotten 6, 7 weist eine genoppte Oberfläche an der zur Anlage an einen Patienten bestimmten Seite der Einlage 5 auf. Zwischen den Enden 1, 2 ist die einem Unterarm eines Patienten zugewandte Oberfläche zudem wellig ausgebildet bzw. gekerbt, so dass die Einlage 5 der Krümmung der konkaven Anlageseite 4 des Formteils 3 folgen kann, ohne, dass sich unerwünschte Druckstellen an dem Unterarm bilden.

Außenseitig an der der Anlageseite 4 gegenüberliegenden Seite weist das Formteil 3 ein Befestigungsmittel 8 auf, mit dem die Epicondylitisspange an einem Unterarm befestigbar ist, wobei das Befestigungsmittel 8 eine um den Unterarm legbare Lasche 9 umfasst, die außenseitig an dem Formteil 3 anklettbar ist.

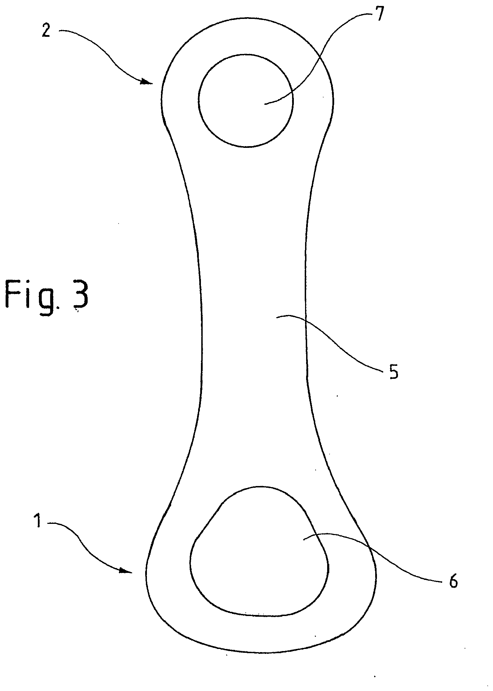

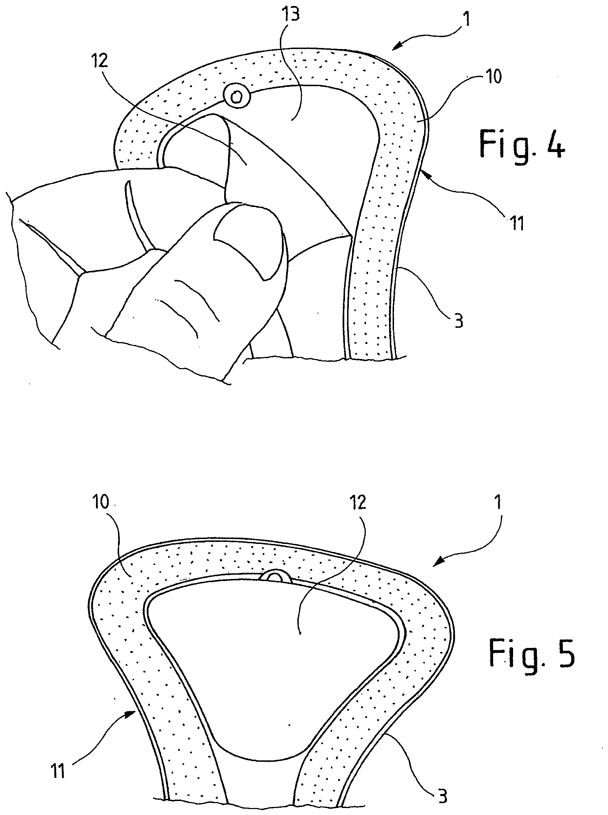

Die unterschiedlich geformten Enden 1, 2 des Formteils 3 und der Einlage 5 werden nochmals aus Figur 3 ersichtlich, in der die Einlage 5 ohne Formteil 3 abgebildet ist. Während die zweite Pelotte 7 der Einlage 5 kreisförmig ausgebildet ist, weist die erste Pelotte 6 eine angenäherte Dreieckform mit ausgerundeten Ecken auf, wobei die ausgerundeten Ecken eine Krümmung aufweisen, die im Wesentlichen der Krümmung des kreisförmigen äußeren Randes der zweiten Pelotte 7 entspricht. Der Anlagebereich 13 des ersten Endes 1 ist dabei um den Abstand der ausgerundeten Ecken zueinander größer als der Anlagebereich des zweiten Endes 2. Zwischen den Enden 1, 2 mit den Pelotten 6, 7 ist die Einlage 5 ebenso wie das Formteil 3 gegenüber den Enden 1, 2 tailliert ausgebildet. An der einem zu behandelnden Unterarm zugewandten Seite weist der taillierte Bereich zudem eine gerippte Oberflächenstruktur quer zur Krümmung des Formteils 3 auf, die eine einfache Biegung der Einlage 5 begünstigt. Der an den Enden 1, 2 um die Pelotten 6, 7 herum ausgebildete Randbereich entspricht in seiner Breite im Wesentlichen der Breite eines Klettstreifens 10 eines Klettverschlusses 11 zur Verbindung von Einlage 5 und Formteil 3, wie er aus Figur 4 bis Figur 7 ersichtlich ist.

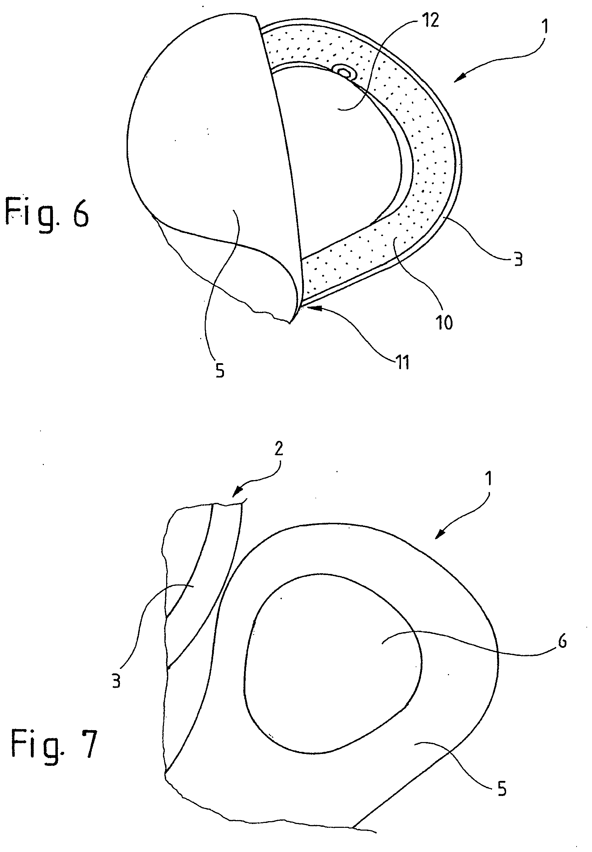

Figur 4 bis Figur 7 zeigen, wie die zusätzliche Pelotte 12 im Anlagebereich 13 zwischen der Einlage 5 und dem Formteil 3 angeordnet wird. Dazu weist das Formteil 3 an dem dargestellten Ende 2 einen Anlagebereich 13 auf, um den herum an einem äußeren Rand der Anlageseite 4 der Klettstreifen 10 des Klettverschlusses 11 angeordnet ist. Die Pelotte 12 wird dann mit einer glatten, ebenen Anlagefläche an den Anlagebereich 13 des Formteils 3 angedrückt, wobei eine Anfangshaftung der Pelotte 12 durch deren blockende Eigenschaften gewährleistet ist. Die an dem Anlagenbereich 13 haftende Pelotte 12 ist in Figur 5 dargestellt, aus der insbesondere die an den Anlagenbereich 13 angepasste Form der Pelotte 12 deutlich wird. Nach dem Platzieren der Pelotte 12 wird dann die Einlage 5 an dem Klettstreifen 10 des Formteil 3 durch Andrücken befestigt. Wie in Figur 5 dargestellt wird die Einlage 5 dabei an einem der Enden 2 beginnend hin zum anderen Ende 1 in Anlage mit dem Formteil 3 gebracht. In Figur 6 liegt die Einlage 5 dann vollständig an dem Formteil 3 an, wobei nur die Enden 1, 2 jeweils einen Klettstreifen 10 aufweisen und der taillierte Bereich im Mittelabschnitt durch die Krümmung des Formteils 3 in Position gehalten ist.

Alle in der vorstehenden Beschreibung und in den Ansprüchen genannten Merkmale sind in einer beliebigen Auswahl mit den Merkmalen des unabhängigen Anspruchs kombinierbar.

## Patentansprüche

1. Epicondylitisspange zur Anlage an einen Unterarm eines Patienten, aufweisend ein zwischen einem ersten und einem zweiten Ende (1, 2) gekrümmtes Formteil (3) mit einer konkaven Anlageseite (4), ein an dem Formteil (3) angebrachtes Befestigungsmittel (8) zum Anlegen der Epicondylitisspange und eine an der Anlageseite (4) angeordnete Einlage (5) mit wenigstens einer in die Einlage (5) eingearbeiteten Pelotte (6, 7), wobei die Einlage (5) lösbar an dem Formteil (3) befestigt ist,
**dadurch gekennzeichnet,**
**dass** zwischen der Einlage (5) und dem Formteil (3) wenigstens ein Anlagebereich (13) für wenigstens eine weitere Pelotte (12) ausgebildet ist.

2. Epicondylitisspange nach Anspruch 1, **dadurch gekennzeichnet, dass** die Einlage (5) und das Formteil (3) über wenigstens einen Klettverschluss (11) miteinander verbunden sind.

3. Epicondylitisspange nach Anspruch 2, **dadurch gekennzeichnet, dass** der Klettverschluss (11) in einem Randbereich des Formteils (2), der Einlage (5) oder des Formteils (3) und der Einlage (5) angeordnet ist.

4. Epicondylitisspange nach Anspruch 3, **dadurch gekennzeichnet, dass** der Anlagebereich (13) für die wenigstens eine weitere Pelotte (12) von dem als Klettverschluss (11) ausgebildeten Randbereich eingefasst ist.

5. Epicondylitisspange nach einem der Ansprüche 1 bis 4, **dadurch gekennzeichnet, dass** wenigstens eines der Enden (1, 2) den Anlagebereich (13) aufweist.

6. Epicondylitisspange nach einem der Ansprüche 1 bis 5, **dadurch gekennzeichnet, dass** beide Enden (1, 2) je einen Anlagebereich (13) aufweisen.

7. Epicondylitisspange nach Anspruch 6, **dadurch gekennzeichnet, dass** das erste Ende (1) einen größeren Anlagebereich (13) als das zweite Ende (2) aufweist.

8. Epicondylitisspange nach einem der Ansprüche 1 bis 7, **dadurch gekennzeichnet, dass** die Einlage (5) und das Formteil (3) nur an den Enden (1, 2) miteinander verbunden sind.

9. Epicondylitisspange nach einem der Ansprüche 1 bis 8, **dadurch gekennzeichnet, dass** das Formteil (3) zwischen den Enden (1, 2) tailliert ausgebildet ist.

10. Epicondylitisspange nach einem der Ansprüche 1 bis 9, **dadurch gekennzeichnet, dass** die Anlageseite (4) des Formteils (3) und die Einlage (5) zueinander kongruente Flächen aufweisen.

11. Epicondylitisspange nach einem der Ansprüche 1 bis 10, **dadurch gekennzeichnet, dass** die Einlage (5) zwischen den Enden (1, 2) eine gewellte Oberflächenstruktur aufweist.

12. Epicondylitisspange nach einem der Ansprüche 1 bis 11, **dadurch gekennzeichnet, dass** das Formteil (3) den wenigstens einen Anlagebereich (13) aufweist.

13. Epicondylitisspange nach einem der Ansprüche 1 bis 12, **dadurch gekennzeichnet, dass** die in die Einlage (5) eingearbeitete Pelotte (6, 7) an einem Ende (1, 2) der Einlage (5) angeordnet ist und zusammen mit der in dem Anlagebereich (13) positionierbaren Pelotte (12) einen gemeinsamen Druckpunkt ausbildet.

14. Epicondylitisspange nach einem der Ansprüche 1 bis 13, **dadurch gekennzeichnet, dass** die den Enden (1, 2) zugeordneten Pelotten (6, 7, 12) von Einlage (5) und Anlagebereich (13) eine an die Breite der Enden (1, 2) angepasste Größe aufweisen.

15. Epicondylitisspange nach einem der Ansprüche 1 bis 14, **dadurch gekennzeichnet, dass** als Befestigungsmittel (8) ein außenseitig an dem Formteil (3) angeordneter Klettverschluss (11) vorgesehen ist.

## Claims

1. An epicondylitis brace for contact with // abutting against a patient's forearm, comprising a curved molded part (3) having a concave contact side (4) between a first end and a second end (1, 2), fastening means (8) attached to the molded part (3) for contacting the epicondylitis brace and an insert (5) which is arranged on the contact side (4) and has at least one skirt (6, 7) incorporated into the insert (5), wherein the insert (5) is releasably attached to the molded part (3),
**characterized in that** at least one contact region (13) for at least one additional skirt (12) is formed between the insert (5) and the molded part (3).

2. The epicondylitis brace according to claim 1,**characterized in that**
the insert (5) and the molded part (3) are connected to one another by at least one Velcro-type closure (11) .

3. The epicondylitis brace according to claim 2,
**characterized in that**
the Velcro-type closure (11) is arranged in a boundary region of the molded part (2), of the insert (5) or of the molded part (3) and the insert (5).

4. The epicondylitis brace according to claim 3,
**characterized in that**
the contact region (13) for the at least one additional skirt (12) is enclosed // framed by the boundary region designed as a Velcro-type closure (11) .

5. The epicondylitis brace according to any one of claims 1 to 4,
**characterized in that**
at least one of the ends (1, 2) has the contact region (13) .

6. The epicondylitis brace according to any one of claims 1 to 5,
**characterized in that**
both ends (1, 2) have one contact region (13) each.

7. The epicondylitis brace according to claim 6,
**characterized in that**
the first end (1) has a larger contact region (13) than the second end (2).

8. The epicondylitis brace according to any one of claims 1 to 7,
**characterized in that**
the insert (5) and the molded part (3) are joined to one another only at the ends (1, 2).

9. The epicondylitis brace according to any one of claims 1 to 8,
**characterized in that**
the molded part (3) is designed with a waste // cinched area between the ends (1, 2).

10. The epicondylitis brace according to any one of claims 1 to 9,
**characterized in that**
the contact side (4) of the molded part (3) and the insert (5) have surfaces that are congruent to one another.

11. The epicondylitis brace according to any one of claims 1 to 10,
**characterized in that**
the insert (5) has a wavy // corrugated surface structure between the ends (1, 2).

12. The epicondylitis brace according to any one of claims 1 to 11,
**characterized in that**
the molded part (3) has the at least one contact region (13).

13. The epicondylitis brace according to any one of claims 1 to 12,
**characterized in that**
the skirt (6, 7) incorporated into the insert (5) is arranged at one end (1, 2) of the insert (5) and together with the skirt (12) that can be positioned in the contact region (13) forms a joint // shared pressure point.

14. The epicondylitis brace according to any one of claims 1 to 13,
**characterized in that**
the skirts (6, 7, 12) assigned to the ends (1, 2) // skirts of the insert (5) and the contact region (13) each have a size adapted to the width of the ends (1, 2) .

15. The epicondylitis brace according to any one of claims 1 to 14,
**characterized in that**
a Velcro-type closure (11) arranged on the outside of the molded part (3) is provided as the fastening means (8) .

## Revendications

1. Orthèse épicondylienne destinée à être appliquée sur un avant-bras d'un patient, comportant une pièce moulée (3) incurvée entre une première et une deuxième extrémités (11, 2), avec une face d'appui (4) concave, un moyen de fixation (8) monté sur la pièce moulée (3), pour l'appui de l'orthèse épicondylienne et un insert (5) placé sur la face d'appui (4), avec au moins une pelotte (6, 7) incorporée dans l'insert (5) l'insert (5) étant fixé de manière amovible sur la pièce moulée (3),
**caractérisée**
**en ce qu'**entre l'insert (5) et la pièce moulée (3) est formée au moins une zone d'application (13) pour au moins une pelotte (12) supplémentaire.

2. Orthèse épicondylienne selon la revendication 1,
**caractérisée en ce que** l'insert (5) et la pièce moulée (3) sont assemblés l'un à l'autre par l'intermédiaire d'au moins une fermeture autoagrippante (11).

3. Orthèse épicondylienne selon la revendication 2,
**caractérisée en ce que** la fermeture autoagrippante (11) est placée dans une zone de bordure de la pièce moulée (2), de l'insert (5) ou de la pièce moulée (3) et de l'insert (5).

4. Orthèse épicondylienne selon la revendication 3,
**caractérisée en ce que** la zone d'application (13) pour l'au moins une pelotte (12) supplémentaire est entourée par la zone de bordure conçue sous la forme d'une fermeture autoagrippante (11).

5. Orthèse épicondylienne selon l'une quelconque des revendications 1 à 4, **caractérisée en ce qu'**au moins l'une des extrémités (1, 2) comporte la zone d'application (13).

6. Orthèse épicondylienne selon l'une quelconque des revendications 1 à 5, **caractérisée en ce que** les deux extrémités (1, 2) comportent chacune une zone d'application (13).

7. Orthèse épicondylienne selon la revendication 6,
**caractérisée en ce que** la première extrémité (1) comporte une zone d'application (13) plus grande que la deuxième extrémité (2).

8. Orthèse épicondylienne selon l'une quelconque des revendications 1 à 7, **caractérisée en ce que** l'insert (5) et la pièce moulée (3) ne sont assemblés l'un à l'autre que sur les extrémités (1, 2).

9. Orthèse épicondylienne selon l'une quelconque des revendications 1 à 8, **caractérisée en ce qu'**entre les extrémités (1, 2), la pièce moulée (3) est conçue en étant taillée.

10. Orthèse épicondylienne selon l'une quelconque des revendications 1 à 9, **caractérisée en ce que** la face d'appui (4) de la pièce moulée (3) et l'insert (5) comportent des surfaces congruentes l'une avec l'autre.

11. Orthèse épicondylienne selon l'une quelconque des revendications 1 à 10, **caractérisée en ce qu'**entre les extrémités (1, 2), l'insert (5) comporte une structure superficielle ondulée.

12. Orthèse épicondylienne selon l'une quelconque des revendications 1 à 11, **caractérisée en ce que** la pièce moulée (3) comporte l'au moins une zone d'application (13) .

13. Orthèse épicondylienne selon l'une quelconque des revendications 1 à 12, **caractérisée en ce que** la pelotte (6, 7) incorporée dans l'insert (5) est placée sur une extrémité (1, 2) de l'insert (5) et en commun avec la pelotte (12) positionnable dans la zone d'application (13), forme un point de pression commun.

14. Orthèse épicondylienne selon l'une quelconque des revendications 1 à 13, **caractérisée en ce que** les pelottes (6, 7, 12) de l'insert (5) et de la zone d'application (13) associées aux extrémités (1, 2) présentent une dimension adaptée à la largeur des extrémités (1, 2).

15. Orthèse épicondylienne selon l'une quelconque des revendications 1 à 14, **caractérisée en ce qu'**en tant que moyen de fixation (8), il est prévu une fermeture autoagrippante (11) placée sur la face extérieure sur la pièce moulée (3).
